Europäisches Patentamt

European Patent Office

Office européen des brevets

(1) Publication number: **0 166 944**
**B1**

(12) **EUROPEAN PATENT SPECIFICATION**

(45) Date of publication of patent specification: 25.04.90

(51) Int. Cl.⁵: **G 01 N 33/84**

(21) Application number: 85106342.0

(22) Date of filing: 23.05.85

(54) Method of releasing protein-bound calcium.

(30) Priority: 25.05.84 US 614251
11.09.84 US 649544

(43) Date of publication of application:
08.01.86 Bulletin 86/02

(45) Publication of the grant of the patent:
25.04.90 Bulletin 90/17

(84) Designated Contracting States:
AT BE CH DE FR GB IT LI LU NL SE

(56) References cited:
US-A-4 382 122

ANALYTICAL CHEMISTRY, vol. 53, no. 13,
November 1981, pages 1970-1974, Easton,
Pennsylv., US; P. ANKER et al.: "Neutral carrier
based ion-selective electrode for the
determination of total calcium in blood serum"

(73) Proprietor: NOVA BIOMEDICAL CORPORATION
200 Prospect Street
Waltham Massachusetts, 02254-9141 (US)

(72) Inventor: Young, Chung Chang
3 Huntington Street
Natick Massachusetts 01760 (US)
Inventor: Mulholland, Linda M.
38 Kinsley Street
Stoughton Massachusetts 02072 (US)

(74) Representative: Heidrich, Udo, Dr. jur., Dipl.-
Phys.
Rechtsanwalt & Patentanwalt Dipl.-Phys. Dr. jur.
U. HEIDRICH Franziskanerstrasse 30
D-8000 München 80 (DE)

Courier Press, Leamington Spa, England.

## Description

This invention relates to the measurement of calcium using ion specific electrodes.

In biological fluids calcium ordinarily exists both in the form of calcium ions ($Ca^{++}$), and calcium complexed with protein and, to a lesser extent, other complexing agents in the fluids. Ion selective calcium electrodes measure only ionized, and not complexed, calcium despite the fact that it would be desirable in some situations to measure, in addition to ionized calcium, "total" calcium, i.e., the sum of ionized and complexed calcium.

It is known that complexed calcium can be released by lowering the pH of the sample containing the complexed calcium. The most efficient releasing of calcium (over 90% calcium released) is observed when a sufficient amount of a strong acid is added to the liquid to lower the pH below 3.0. Such a drastic lowering of pH, however, is inconsistent with optimal operation of conventional calcium electrodes.

In general, the present invention features a method using a releasing agent for releasing, as ionized calcium, complexed calcium, in a liquid sample for a measurement of such ionized calcium, together with any ionized calcium originally present in the sample, using a calcium ion specific electrode.

The releasing reagent includes formic acid and formate salt (e.g., the sodium salt), in concentrations sufficient to render the pH of the sample, when mixed with the reagent, in the range of 4.0—5.5, most preferable 4.5.

The formic acid and formate salt together cause the release of calcium in biological samples. For samples, e.g., spinal fluid, in which complexed calcium is bound not to protein but to other constituents of the liquid, the releasing reagent need not contain ingredients in addition to formic acid and formate salt. For other samples, e.g. serum, which do not contain appreciable amounts of protein, it is desirable that the releasing reagent contain an additional component which inhibits precipitation of the protein in the sample. Preferably, this precipitation inhibiting component is the salt of a strong organic acid, e.g., a haloacetate salt such as a monochloroacetate or trifluoroacetate salt. Of these latter two, trifluoroacetate salt is most preferred because of its superior long-term stability.

In particular embodiments of the invention, the concentration of formic acid is between 0.105 Molar and 0.195 Molar, and is preferably 0.15 Molar; and the concentration of formate salt is between 0.21 Molar and 0.39 Molar, and is preferably 0.30 Molar.

When the releasing reagent contains monochloroacetate salt, this component is preferably present in a concentration of between 0.385 Molar and 0.715 Molar, most preferably 0.55 Molar. When trifluoroacetate is present, it is preferably at a concentration of between 0.225 Molar and 0.416 Molar, most preferably 0.32 Molar.

In other particular embodiments the method is for use in apparatus in which the reagent is added to the sample at the same time as a reference solution is added to the sample; in this instance the reagent includes the reference solution, which is preferably ammonium chloride, preferably present in a concentration of between 1.5 Molar and 3.0 Molar, most preferably 2.0 Molar.

The releasing method of the invention provides efficient (greater than 99%) release of complexed calcium while avoiding detrimental extreme acidic conditions.

Furthermore, the reagent, including or not including a reference solution, minimizes liquid junction and interference effects.

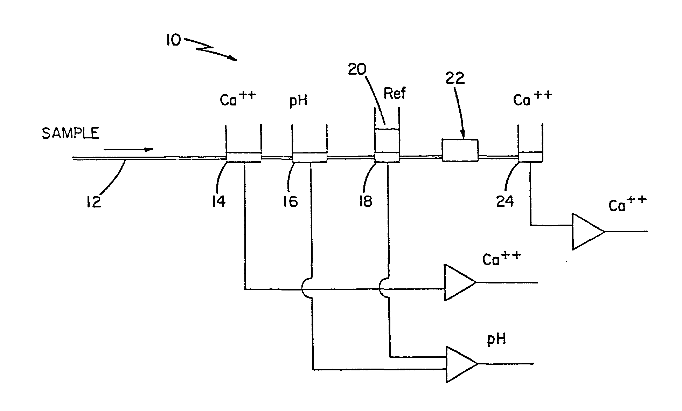

Other objects, features and advantages of this invention will be apparent to those skilled in the art from the following detailed description of a preferred embodiment thereof, taken together with the accompanying drawing, in which:

The Figure is a schematic representation of electrode apparatus employing the method of the invention. Much of the structure and operation of the apparatus is as described in US—A—4,314,895.

Referring to the Figure, in the preferred embodiment of the present invention the releasing reagent entering the system via line 20 has one of the following two compositions:

2.0  Molar ammonium chloride
0.55 Molar monochloroacetate (sodium salt)
0.15 Molar formic acid
0.30 Molar formate (sodium salt) or

2.0  Molar ammonium chloride
0.32 Molar trifluoroacetate (sodium salt)
0.15 Molar formic acid
0.30 Molar formate (sodium salt).

A sample (e.g. urine, blood, or spinal fluid) enters the electrode apparatus via line 12 and flows past conventional calcium ion specific electrode 14 and conventional pH electrode 16, both of which measure their respective ions with reference to conventional reference electrode 18. As the sample flows past reference electrode 18, the releasing reagent of the invention is injected into the system via line 20 and mixes with the sample in mixer 22.

The reagent causes the release of the complexed calcium in the sample so that the released calcium ions, together with calcium ions originally present in the sample, can be measured by the second calcium ion specific electrode 24 (which also uses reference electrode 18) to give a measurement of total calcium.

The illustrated system measures pH, ionized calcium, and total calcium, while maintaining the pH of the sample and reagent mixture at 4.5. Despite the fairly high, system-compatible pH, calcium releasing is generally close to 100%, providing a relatively accurate total calcium measurement. Since the system also measures originally ionized calcium, complexed calcium can be estimated by subtracting ionized from total calcium.

Other embodiments of the invention are with the following claims. For example, the concentrations of the reagent components can vary within the ranges given above. The pH of the reagent, prior to being mixed with the sample, is not much affected by variations within the stated ranges, and is generally 4.0 for all formulations. However, the concentration of reagent components, particularly of the formic acid, does affect the pH of the reagent/sample mixture; a greater concentration of acid will produce a mixture with a lower pH. The pH of the mixture, as mentioned above, can range from 4.0 to 5.5; pH values outside this range at the high end will have inadequate calcium releasing power, while values outside the range at the low end can be harmful to electrode operation.

The concentration of ammonium chloride also has an effect on the operation of the system. Although the main function of ammonium chloride is as a reference solution for the reference electrode, ammonium chloride also aids, to a minor extent, the releasing of complexed calcium. Ammonium chloride concentrations outside the stated range at the high end of the range have the potential of causing interference, while concentrations outside the range at the low end can, in a flow-through system such as that illustrated in the Figure, cause undesirable liquid junction effects.

As mentioned above, the reagent of the invention can be used in systems other than that illustrated in the Figure. For example, the reagent can be used in a system in which a sample stream is split into two streams, one of which flows through a calcium ion specific electrode for measurement of ionized calcium, and the other of which is mixed with the reagent and then flows through a calcium ion specific electrode for measurement of total calcium. In this type of system, a reference solution such as ammonium chloride is not a necessary reagent component, although it can still be used to aid in calcium releasing.

## Claims

1. Method of releasing protein-bound calcium
—as ionized calcium in a liquid sample,
—for measurement of said ionized calcium,
—together with any ionized calcium originally present in said sample,
—using a calcium ion specific electrode, characterized in that
—said sample is mixed with
—a releasing agent comprising
—a formate salt and
—formic acid
—at concentrations sufficient to render the pH of the mixture of said sample and said reagent
—in the range of 4.0—5.5.
2. Method of claim 1, characterized by
—0.21—0.39 Molar formate salt and
—0.105—0.195 Molar formic acid.
3. Method of claim 2, characterized by
—0.30 Molar formate salt and
—0.15 Molar formic acid.

4. Method of any of the preceding claims, wherein said sample contains protein, characterized in that
—said releasing agent comprises
—a component
—capable of inhibiting precipitation of said protein in said sample.
5. Method of claim 4, characterized in that
—said precipitation inhibiting component comprises
—a salt of a strong organic acid.
6. Method of claim 5, characterized in that
—said strong organic acid comprises
—an acetate salt.
7. Method of claim 6, characterized in that
—said acetate salt comprises
—monochloroacetate salt.
8. Method of claim 7, characterized by
—0.385—0.715 Molar monochloroacetate salt.
9. Method of claim 8, characterized in that
—said releasing agent comprises
—0.55 Molar monochloroacetate.
10. Method of claim 6, characterized in that
—said acetate salt comprises
—trifluoroacetate salt.
11. Methof of claim 10, characterized by
—0.224—0.416 Molar trifluoroacetate salt.
12. Method of claim 11, characterized in that
—said releasing agent comprises
—0.32 Molar trifluoroacetate.
13. Method of any of the preceding claims, characterized in that
—said releasing agent comprises
—ammonium chloride.
14. Method of claim 13, characterized by
—1.5—3.0 Molar ammonium chloride.
15. Method of claim 14, characterized by
—2.0 Molar ammonium chloride.
16. Method of claim 13, 14 or 15, wherein said reagent is for use in apparatus which
—said reagent is added to said sample
—at the same time a reference solution is added to said sample, characterized in that
—said releasing agent comprises
—said reference solution.

## Patentansprüche

1. Verfahren zum Abtrennen von proteingebundenem Calcium
—als ionisiertes Calcium in einer Flüssigkeits-Probe
—zum Messen des ionisierten Calciums
—zusammen mit allem ursprünglich in der Probe vorhandenen ionisierten Calcium
—mittels einer Calcium-Ionen-selektiven Elektrode, gekennzeichnet dadurch, daß
—die Probe gemischt wird mit
—einem Trennmittel, enthaltend
—Formiat-Salz und
—Ameisensäure
—in ausreichender Konzentration für die Einstellung des pH-Werts des Gemisches von Probe und Trennmittel
—auf 4,0—5,5.

2. Verfahren nach Anspruch 1, gekennzeichnet durch
—0,21—0,39 M Formiat-Salz und
—0,105—0,195 M Ameisensäure.

3. Verfahren nach Anspruch 2, gekennzeichnet durch
—ca. 0,30 M Formiat-Salz und
—ca. 0,15 M Ameisensäure.

4. Verfahren nach einem der vorhergehenden Ansprüche, wobei die Probe Protein enthält, gekennzeichnet dadurch, daß
—das Trennmittel
—eine Komponente
—zum Hemmen der Protein-Fällung in der Probe enthält.

5. Verfahren nach Anspruch 4, gekennzeichnet dadurch, daß
—die Fällungs-Hemm-Komponente
—ein Salz einer starken organischen Säure enthält.

6. Verfahren nach Anspruch 5, gekennzeichnet dadurch, daß
—die starke organische Säure
—ein Acetate-Salz enthält.

7. Verfahren nach Anspruch 6, gekennzeichnet dadurch, daß
—das Acetat-Salz
—Monochloroacetat-Salz enthält.

8. Verfahren nach Anspruch 7, gekennzeichnet durch
—0,385—0,715 M Monochloroacetat-Salz.

9. Verfahren nach Anspruch 8, gekennzeichnet dadurch, daß
—das Trennmittel
—ca. 0,55 M Monochloroacetat enthält.

10. Verfahren nach Anspruch 6, gekennzeichnet dadurch, daß
—das Acetat-Salz
—Trifluoroacetat-Salz enthält.

11. Verfahren nach Anspruch 10, gekennzeichnet durch
—0,224—0,416 M Trifluoroacetat-Salz.

12. Verfahren nach Anspruch 11, gekennzeichnet dadurch, daß
—das Trennmittel
—ca. 0,32 M Trifluoroacetat enthält.

13. Verfahren nach einem der vorhergehenden Ansprüche, gekennzeichnet dadurch, daß
—das Trennmittel
—Ammoniumchlorid enthält.

14. Verfahren nach Anspruch 13, gekennzeichnet durch
—1,5—3,0 M Ammoniumchlorid.

15. Verfahren nach Anspruch 14, gekennzeichnet durch
—ca. 2,0 M Ammoniumchlorid.

16. Verfahren nach Anspruch 13, 14 oder 15, wobei das Trennmittel für eine Einrichtung vorgesehen ist, in der
—der Probe das Trennmittel
—gleichzeitig mit einer Bezugs-Lösung zugegeben wird, gekennzeichnet dadurch, daß
—das Trennmittel
—die Bezugs-Lösung enthält.

**Revendications**

1. Procédé pour la libération de calcium lié à la protéine
—en tant que calcium ionisé dans un échantillon liquide,
—pour la mesure du calcium ionisé,
—conjointement avec tout calcium ionisé présent à l'origine dans l'échantillon,
—en utilisant une électrode spécifique aux ions calcium, caractérisé en ce que
—l'échantillon est mélangé avec
—un agent réactif de libération comprenant
—du sel de formate et
—de l'acide formique
—à des concentrations suffisantes pour amener le pH du mélange de l'échantillon et de l'agent réactif
—dans la plage de 4,0—5,5.

2. Procédé selon la revendication 1, caractérisé par
—~0,21—0,39 molaire de sel de formate et
—~0,105—0,195 molaire d'acide formique.

3. Procédé selon la revendication 2, caractérisé par
—0,30 molaire de sel de formate et
—0,15 molaire d'acide formique.

4. Procédé selon l'une quelconque des revendications précédentes, dans lequel l'échantillon contient la protéine, caractérisé en ce que
—l'agent de libération comprend
—un composant
—capable d'inhiber la précipitation de la protéine dans l'échantillon.

5. Procédé selon la revendication 4, caractérisé, en ce que
—le composant inhibant la précipitation comprend
—un sel d'un acide organique fort.

6. Procédé selon la revendication 5, caractérisé en ce que
—l'acide organique fort comprend
—un sel d'acétate.

7. Procédé selon la revendication 6, caractérisé en ce que
le sel d'acétate comprend
—du sel de monochloracétate.

8. Procédé selon la revendication 7, caractérisé par
—0,385—0,715 molaire de sel de monochloracétate.

9. Procédé selon la revendication 8, caractérisé en ce que
—l'agent de libération comprend
—~0,55 molaire de monochloracétate.

10. Procédé selon la revendication 6, caractérisé en ce que
—le sel d'acétate comprend
—du sel de trifluoroacétate.

11. Procédé selon la revendication 10, caractérisé par
—0,224—0,416 molaire de sel de trifluoroacétate.

12. Procédé selon la revendication 11, caractérisé en ce que

—l'agent de libération comprend
~—0,32 molaire de trifluoroacétate.

13. Procédé selon l'une quelconque des revendications précédentes, caractérisé en ce que
—l'agent de libération comprend
—du chlorure d'ammonium.

14. Procédé selon la revendication 13, caractérisé par
—1,5—3,0 molaire de chlorure d'ammonium.

15. Procédé selon la revendication 14, caractérisé par
—~2,0 molaire de chlorure d'ammonium.

16. Procédé selon la revendication 13, 14 ou 15, dans lequel l'agent réactif est destiné à l'utilisation dans un appareil dans lequel
—l'agent réactif est ajouté à l'échantillon
—en même temps, une solution de référence est ajoutée à l'échantillon, caractérisé en ce que
—l'agent de libération comprend
—la solution de référence.